# EUROPEAN PATENT SPECIFICATION

(11) **EP 3 466 936 B1**
(45) Date of publication and mention of the grant of the patent: **24.06.2020**
(21) Application number: 17739690.0
(22) Date of filing: 29.05.2017
(51) Int. Cl.: C07D 307/93

(54) **EXTRACTION PROCESSES FOR CYNAROPICRIN PRESENT IN THE LEAVES OF CYNARA CARDUNCULUS L.**
VERFAHREN ZUR EXTRAKTION VON CYNAROPICRIN AUS BLÄTTERN VON CYNARA CARDUNCULUS L.
PROCÉDÉS D'EXTRACTION DE LA CYNAROPICRINE PRÉSENTE DANS LES FEUILLES DE CYNARA CARDUNCULUS L.

(30) Priority: 27.05.2016 PT 2016109415
(43) Date of publication of application: 10.04.2019
(73) Proprietor: Cebal - Centro De Biotecnologia Agrícola E Agro-Alimentar Do Alentejo, 7801-908 Beja (PT); Amoreira Sousa Silva Brás, Teresa Isabel, 7600-017 Aljustrel (PT); Pereira Duarte Ricardo, Maria De Fátima, 7800-073 Beja (PT); Graça Neves, Luísa Alexandra, 2820-142 Charneca Da Caparica (PT); Serejo Goulão Crespo, João Paulo, 1300-319 Lisboa (PT)
(72) Inventor: AMOREIRA SOUSA SILVA BRÁS, Teresa Isabel, 1300-319 Lisboa (PT); PEREIRA DUARTE RICARDO, Maria De Fátima, 1300-319 Lisboa (PT); GRAÇA NEVES, Luísa Alexandra, 1300-319 Lisboa (PT); SEREJO GOULÃO CRESPO, João Paulo, 1300-319 Lisboa (PT)
(74) Representative: Ferreira, Maria Silvina
(86) International application number: PCT/IB2017/053152
(87) International publication number: WO 2017/203498

(56) References cited:
- RAMOS P A B ET AL: "Lipophilic Extracts of Cynara cardunculus L. var. altilis (DC): A Source of Valuable Bioactive Terpenic Compounds", JOURNAL OF AGRICULTURAL AND FOOD CHEMISTRY, vol. 61, no. 35, 4 September 2013 (2013-09-04), pages 8420-8429, XP055399840, ISSN: 0021-8561, DOI: 10.1021/jf402253a cited in the application
- ELJOUNAIDI K ET AL: "Accumulation of cynaropicrin in globe artichoke and localization of enzymes involved in its biosynthesis", PLANT SCIENCE, vol. 239, October 2015 (2015-10), pages 128-136, XP029277002, ISSN: 0168-9452, DOI: 10.1016/J.PLANTSCI.2015.07.020

## Description

### Technical Field

The present application relates to methods for the extraction of the sesquiterpene lactone cynaropicrin, present in leaves of *Cynara cardunculus.* The extraction is carried out using four extraction methods, solid-liquid batch extraction, ultrasounds, microwaves, and extraction by pressurized liquid and pure solvents, such as ethanol and ethyl acetate, and a solvent mixture, such as ethanol/water mixture.

### Background of the Invention

Belonging to the sesquiterpene lactone family, cynaropicrin is primarily recognized by the bitter taste it gives to food. Present in greater abundance in leaves of the family Asteraceae, it can be found in the commercially known artichoke (*Cynara cardunculus* var. *Scolymus* L.).

Based on its presence in *Cynara scolymus*, several previous studies have demonstrated its potential for application in the food, pharmaceutical and nutraceutical industries.

Originating from leaves or aerial parts of the *Cynara scolymus* plant, several studies have already been done and/or patented in order to demonstrate its potential for application, such as:
Assessment of ability to reduce cholesterol. For this, artichoke leaves were subjected to extraction techniques, confirming their potential for application in cholesterol reduction (Fritsche, Beindorff *et al*., 2002). The present invention is distinct from the cited study for using leaves of *Cynara cardunculus* and not commercially available artichoke products.

Study on the anti-hyperlipidemic potential of sesquiterpene lactones. With a study published in 2008, Shimoda *et al*. (Shimoda, Ninomiya *et al*., 2003) have demonstrated anti-hyperlipidemic potential, namely of cynaropicrin, from *Cynara scolymus* L., using a fractionated methanol extract to obtain the cynaropicrin-containing fraction, whereas the present invention does not use additional extraction steps, obtaining a fraction rich in cynaropicrin with a single extraction step.

Determination of antispasmodic activity. Emendörfer *et al*. (Emendörfer, Emendörfer *et al*., 2005) have demonstrated antispasmodic activity of extract fractions, and in particular of cynaropicrin, from *Cynara scolymus*, distinct from the present invention by the use of extract fractionation and a long extraction time (7 days), therefore becoming a time consuming and expensive process, as opposed to the present invention.

Despite a high correlation between the descriptions of the biological potential of cynaropicrin and the extracts derived from the *Cynara scolymus* plant, also cynaropicrin derived from other plants, or even the synthetic form, has been previously studied, showing its application potential, namely in response to anti-inflammatory processes (Cho, Baik *et al*., 2000), and its cytotoxic and pro-apoptotic activity in cancer cells (Cho, Kim *et al*., 2004), with very positive and satisfactory results in both settings. Present in other species of the family Astearecae, the extraction of cynaropicrin has been studied based on different extraction techniques. In 2013, a chemical characterization study of the *Cynara cardunculus* var. *Altilis* plant was published, where for the first time cynaropicrin was quantified in this *Cynara cardunculus* variety (Ramos, Guerra et *al.* 2013). This invention has used a long extraction time of 7 hours and the use of environmentally aggressive solvents. The present invention can be distinguished by the use of environmentally friendly solvents, reduced extraction times and yield of higher cynaropicrin concentrations (40 mg/g dry weight *versus* 55 mg/g dry weight). More recently, the same authors have demonstrated the potential of said sesquiterpene lactone in reducing cell viability of breast cancer cells with a triple-negative phenotype (Ramos, Guerra et al., 2014) .

Despite the well-known biological potential of cynaropicrin, no processes have been developed to obtain higher concentrations of cynaropicrin from leaves of the *Cynara cardunculus* plant.

In order to understand and expand the potential of cynaropicrin application, either in terms of biological potential, and consequently therapeutic, or for food applications, the extraction methods previously published by several authors aimed only at obtaining extract fractions (S) containing cynaropicrin, not having the maximization of the cynaropicrin extraction as a central goal. The following currently published extraction methods can be found:
In order to study the healing properties of *Cynara scolymus* L. extracts, extraction was carried out using ethanol solutions in solid-liquid batch mode, in a proportion of 45% ethanol, obtaining a composition with 1% of cynaropicrin (Ishida, Kojima *et al*., 2010), as opposed to extracts obtained with the present invention, where pure solvents or an ethanol/water mixture at 40% ethanol are used, obtaining an average of 27% of cynaropicrin depending on the selected extraction method.

In order to evaluate its potential in cholesterol reduction, artichoke leaves were submitted to solid-liquid batch extraction processes, also known as maceration, and purification of their bioactive compounds, namely cynaropicrin, using purification and isolation processes by liquid chromatography (Sephadex) (Fritsche, Beindorff et al., 2002), where isolation is not necessary in the present invention to obtain an extract rich in cynaropicrin.

In the described art, in EP 2379092B1, aerial parts of *Cynara scolymus* were subjected to extraction using a heating mantle percolator, with an extraction temperature of 70 °C, and with 70% ethanol as an extraction solvent, obtaining at the end of the process 10% to 13% of cynaropicrin (Bombardelli 2013). In addition to obtaining higher concentrations of cynaropicrin, lower temperatures (between 30 and 60 °C) are used in the present invention.

More recently, capitula of *Cynara scolymus* plant were also subjected to extraction techniques with ethanol/water mixtures in a 7:3 ratio v/v, having in its final composition 1% of cynaropicrin (Bombardelli, Fontana *et al*. 2008), thereby obtaining significantly lower values as compared to the values obtained using the methods described in the present patent application.

In 2013 a characterization study of the *Cynara cardunculus* var. *Altilis* plant was published, where leaf extraction was carried out using Soxhlet extraction, with an extraction time of 7 h having cynaropicrin quantified for the first time in this variety of the *Cynara cardunculus* plant (Ramos, Guerra et al., 2013). The proposed invention differs from the invention of Ramos *et al*. in the use of extraction methods with an extraction time of less than 7 h and in obtaining higher percentages of cynaropicrin.

However, for the extraction of the sesquiterpene lactone cynaropicrin, only maceration (solid-liquid batch) stands out, with solvents such as methanol or ethanol/water mixture, being mostly followed by purification techniques in order to separate the desired fractions.

The present patent application discloses new methods of extracting the sesquiterpene lactone cynaropicrin, present in the leaves of the *Cynara cardunculus* L. plant, such as solid-liquid batch, ultrasounds, microwaves and solvent by pressurized liquid, using different extraction solvents, ethanol, ethanol/water and ethyl acetate. With the methodology described in this patent application for extraction of cynaropicrin, a yield increase and a reduction in the extraction time are achieved as compared to the methodology of the prior art.

### Summary of the Invention

The present patent application describes novel methods for the extraction of the sesquiterpene lactone cynaropicrin present in the leaves of *Cynara cardunculus.*

In one embodiment the method of extracting cynaropicrin from leaves of *Cynara cardunculus* comprises the following steps:
- lyophilisation and grinding of *Cynara cardunculus* leaves, with a particle size of less than 400 µm;
- selecting a solvent in its pure state or in a mixture;
- extraction of the lyophilised biomass with the solvent selected in the previous step in a closed container protected from light.

In another embodiment, the solvent used in the method for extraction of cynaropicrin is ethanol, ethyl acetate or an ethanol/water mixture.

In one embodiment, the container is subjected to the action of bath ultrasound equipment or with a probe during the extraction step. In another embodiment, the extraction step has a duration of between 15 and 240 minutes, takes place at a temperature between 25 and 60 °C and at an ultrasonic power between 5 and 100 kHz.

In one embodiment, the container is subjected to the action of stirring equipment during the extraction step. In another embodiment, the extraction step has a duration of between 15 and 240 minutes, takes place at a temperature between 25 and 60 °C and at a stirring between 300 and 900 rotations per minute.

In one embodiment, the container is subjected to the action of ultrasound equipment during the extraction step. In another embodiment, the extraction step has a duration of between 5 and 120 minutes, takes place at a temperature between 25 and 80 °C and at a microwave power between 500 and 2000 W.

In one embodiment, the container is subjected to the action of pressurized liquid extraction equipment during the extraction step. In another embodiment, the extraction step takes place at a temperature between 25 and 150 °C and over 1 to 5 extraction cycles.

In one embodiment, the biomass/solvent ratio is between 1/4 and 1/100.

In another embodiment, the ethanol/water ratio is 40/60.

### Detailed Description of the Invention

The present patent application relates to novel methods for the extraction of the sesquiterpene lactone cynaropicrin, present in leaves of *Cynara cardunculus.* The extraction is carried out using four methods of extraction, solid-liquid batch extraction, ultrasounds, microwaves and extraction by pressurized liquid and pure solvents, such as ethanol and ethyl acetate, and a solvent mixture, such as an ethanol/water mixture. After the extraction process, no additional extraction and/or purification steps are required.

### Collection and storage

Leaves of *Cynara cardunculus* are harvested fresh before the summer high temperatures in order to avoid the presence of dry biomass. After collecting fresh material, it is immediately lyophilised in any commercially available lyophiliser for the removal of water for 5 to 7 days and/or until a constant mass value is obtained, indicative of complete removal of the moisture present in the fresh leaves. After obtaining a constant mass value, the material is grounded in a knife mill equal to those commercially available, with a grain size of less than 400 µm. After grinding, the material is vacuum packed (optional) in order to avoid subsequent water absorption. This set of processes, from collection to storage, allows for the material to be stored at room temperature, with a reduced storage volume and without deterioration of its compounds of interest.

### Extraction Methods

The following methodology is used for extraction of the sesquiterpene lactone cynaropicrin.

### Ultrasonic extraction

For ultrasonic extraction, the following pure solvents or solvent mixtures can be used as extraction solvents: ethanol, ethyl acetate, and ethanol/water. The extraction procedure aims at placing together, in a closed container protected from light, subjected to ultrasound, bath, probe or similar equipment, the lyophilised biomass and a solvent or mixture of solvents, at a particular biomass/solvent ratio, during a particular period of time, at a particular temperature and at a particular ultrasonic power value. For this procedure, any ultrasonic wave emission device can be used, from probes to baths, with no need for specification. In the present invention, the extraction time is between 15 minutes and 4 hours, preferably between 20 and 90 minutes, the extraction temperature is between 25 °C and 60 °C, preferably between 40 and 60 °C, and the ultrasonic power value is between 5 and 100 kHz, preferably between 10 and 30 kHz. As a biomass/solvent ratio, it has a ratio between 1/4 and 1/100, preferably between 1/10 and 1/50. After extraction, the supernatant is quantified by high-pressure liquid chromatography (HPLC) and results are expressed as mg compound/g lyophilised weight and % of cynaropicrin in the extract.

### Solid-liquid batch extraction

For solid-liquid batch extraction, the following pure solvents or solvent mixtures can be used as extraction solvents: ethanol, ethyl acetate, ethanol/water. The extraction procedure is aimed at placing together, in a closed container and protected from light, with stirring and heating, the lyophilised biomass and the solvent or mixture of solvents, at a particular biomass/solvent ratio, for a particular period of time, at a particular temperature and at a particular stirring rate. For this procedure, any heating and stirring device can be used, with no need for specification. In the present invention, the extraction time is between 20 minutes and 4 hours, preferably between 30 and 90 minutes, the extraction temperature is between 25 °C and 60 °C, preferably between 30 and 50 °C and the stirring value is between 300 and 900, preferably between 400 and 700 rotations per minute. As a biomass/solvent ratio, it has a ratio between 1/4 and 1/100, with 1/10 and 1/50 being preferred. After extraction, the supernatant is quantified by high-pressure liquid chromatography (HPLC) and results are expressed as mg compound/g lyophilised weight and % of cynaropicrin in the extract.

### Microwave extraction

For microwave extraction, the following pure solvents or solvent mixtures can be used as extraction solvents: ethanol, ethyl acetate, and ethanol/water. The extraction procedure is aimed at placing together in a closed container and protected from light, subject to suitable microwave equipment, the lyophilised biomass and the solvent or solvent mixture at a particular biomass/solvent ratio, for a particular period of time, at a particular temperature and at a particular microwave power value. For this procedure, any commercially available microwave digestion device can be used, with no need for specification. In the present invention, the extraction time is between 5 minutes and 2 hours, preferably between 15 and 60 minutes, the extraction temperature is between 25 °C and 80 °C, preferably between 30 and 65 °C, and the value of microwave power is between 500 and 2000 W, preferably between 700 and 1500 W. As a biomass/solvent ratio, it has a ratio between 1/4 and 1/100, with 1/10 and 1/50 being preferred. After extraction, the supernatant is quantified by high-pressure liquid chromatography (HPLC) and results are expressed as mg compound/g lyophilised weight and % of cynaropicrin in the extract.

### Pressurized liquid solvent extraction

For pressurized liquid solvent extraction, the following pure solvents and solvent mixtures can be used as extraction solvents: ethanol, ethyl acetate, and ethanol/water. The extraction procedure is aimed at placing together, in a closed container protected from light, subject to proper equipment for pressurized liquid solvent extraction, the lyophilised biomass and the solvent or solvent mixture at a particular biomass/solvent ratio, at a particular temperature and with a particular number of extraction cycles. For this procedure, any commercially available pressurized liquid solvent extraction equipment can be used, with no need for specification. In the present invention, an extraction temperature is presented, a temperature between 25 and 150 °C, preferably between 30 and 90 °C, and extraction cycles between 1 and 5. As a biomass/solvent ratio, it has a ratio between 1/4 and 1/100, preferably between 1/10 and 1/60. After extraction, the supernatant is quantified by high-pressure liquid chromatography (HPLC) and results are expressed as mg compound/kg lyophilised weight and % of cynaropicrin in the extract.

### Examples

### Ultrasonic extraction:

Ultrasonic extraction with a solid-liquid ratio of 1/16, using ethyl acetate as an extraction solvent, an extraction temperature of 40 °C, an ultrasonic power of 42 kHz, and an extraction time of 60 minutes. The results obtained have shown the attainment of an extract with a concentration of 46 mg of cynaropicrin/g of lyophilisate, 38% in extract base.

### Pressurized liquid solvent extraction:

Pressurized liquid solvent extraction with a solid-liquid ratio of 1/16, using ethanol as an extraction solvent, an extraction temperature of 40 °C and two extraction cycles. The results obtained have shown the attainment of an extract with a concentration of 50 mg of cynaropicrin/g of lyophilisate, and 21% of cynaropicrin in the extract.

### Solid-liquid batch extraction:

Solid-liquid batch extraction with a solid-liquid ratio of 1/16, using ethanol as an extraction solvent, an extraction temperature of 40 °C, stirring at 700 rotations per minute and an extraction time of 60 minutes. The results obtained have shown the attainment of an extract with a concentration of 57 mg of cynaropicrin/g of lyophilisate weight, and 27% of cynaropicrin in the extract.

### Microwave extraction:

Microwave extraction with a solid-liquid ratio of 1/16, using the ethanol/water mixture as an extraction solvent, an extraction temperature of 40 °C, a microwave power of 1000 W and an extraction time of 15 minutes. The results obtained have shown the attainment of an extract with a concentration of 18,51 mg of cynaropicrin/g of lyophilisate, and 11% of cynaropicrin in the extract.

With the methods and solvents disclosed in this patent application, novel methods of extracting cynaropicrin present in the leaves of *Cynara cardunculus* are achieved.

With the methods and solvents disclosed herein, it is possible to extract sesquiterpene lactone from all varieties of *Cynara cardunculus.*

With the present method of solid-liquid batch extraction or maceration, higher values of cynaropicrin are obtained as compared to those obtained in other prior art methods, using different pure solvents and different ethanol/water ratios, as well as lower temperatures, which leads to lower energy costs.

It is important to note that, unlike what happens in the present methodology, ethyl acetate is not normally used for the first extraction phase and is always used as a purification solvent in later phases. With the methodology disclosed in this patent application, it is possible to obtain higher percent values of cynaropicrin than previously published.

The present description is, naturally, not in any way restricted to the embodiments presented herein and a person of ordinary skill in the art may foresee many possibilities of modifying it without departing from the general idea as defined in the claims. The preferred embodiments described above are obviously combinable with each other. The following claims further define preferred embodiments.

### References

Bombardelli, E. (2013). "Compositions comprising a lipophilic extract of *Zingiber officinale* and an extract of *Cynara scolymus*", EP23709092B2.
Bombardelli, E., *et al.* (2008). "*Cynara scolymus* extracts and compositions containing them", EP1967199A1.
Cho, J. Y., et al. (2000). "In vitro anti-inflammatory effects of cynaropicrin, a sesquiterpene lactone, from Saussurea lappa" European journal of pharmacology 398(3): 399-407.
Cho, J. Y., et al. (2004). "Cytotoxic and pro-apoptotic activities of cynaropicrin, a sesquiterpene lactone, on the viability of leukocyte cancer cell lines" European journal of pharmacology 492(2): 85-94.
Emendörfer, F., et al. (2005). "Antispasmodic activity of fractions and cynaropicrin from Cynara scolymus on guinea-pig ileum" Biological and Pharmaceutical Bulletin 28(5): 902-904.
Fritsche, J., et al. (2002). "Isolation, characterization and determination of minor artichoke (Cynara scolymus L.) leaf extract compounds" European Food Research and Technology 215(2): 149-157.
Ishida, K., et al. (2010). "Effects of artichoke leaf extract on gastric mucosal injury in rats" Biological and Pharmaceutical Bulletin 33(2): 223-229.
Ramos, P., et al. (2014). "Antitumoral and antioxidant activities of lipophilic and phenolic extracts from Cynara cardunculus L. var. Altilis (DC)" Planta Medica 80(16): P1L16.
Ramos, P.A., et al. (2013). "Lipophilic extracts of Cynara cardunculus L. var. Altilis (DC): a source of valuable bioactive terpenic compounds" Journal of agricultural and food chemistry 61(35): 8420-8429.
Shimoda, H., et al. (2003). "Anti-hyperlipidemic sesquiterpenes and new sesquiterpene glycosides from the leaves of artichoke (Cynara scolymus L.): structure requirement and mode of action" Bioorganic & medicinal chemistry letters 13(2): 223-228.

## Claims

1. A method for extracting cynaropicrin from leaves of *Cynara cardunculus,* **characterized in that** it comprises the following steps:
- lyophilisation and grinding of leaves of *Cynara cardunculus,* with a particle size of less than 400 µm;
- selecting a solvent from ethanol or ethyl acetate in its pure state or in a mixture;
- extraction of the lyophilised biomass with the solvent selected in the previous step in a closed container and protected from light.

2. A method for extracting cynaropicrin according to claim 1, wherein the solvent mixture is an ethanol/water mixture.

3. A method for extracting cynaropicrin according to any one of claims 1 or 2, wherein in the extraction step the container is subjected to the action of a bath or probe ultrasound equipment.

4. A method for extracting cynaropicrin according to any claim 3, wherein the extraction step has a duration of between 15 and 240 minutes, takes place at a temperature between 25 and 60 °C and at an ultrasonic power between 5 and 100 kHz.

5. A method for extracting cynaropicrin according to any one of claims 1 or 2, wherein in the extraction step the container is subjected to the action of a stirring equipment.

6. A method for extracting cynaropicrin according to claim 5, wherein the extraction step has a duration of between 15 and 240 minutes, takes place at a temperature between 25 and 60 °C and with a stirring of between 300 and 900 rotations per minute.

7. A method for extracting cynaropicrin according to any one of claims 1 or 2, wherein in the extraction step the container is subjected to the action of a microwave equipment.

8. A method for extracting cynaropicrin according to claim 7, wherein the extraction step has a duration of between 5 and 120 minutes, takes place at a temperature between 25 and 80 °C and at a microwave power of between 500 and 2000 W.

9. A method for extracting cynaropicrin according to any one of claims 1 or 2, wherein in the extraction step the container is subjected to the action of a pressurized liquid extraction equipment.

10. A method for extracting cynaropicrin according to claim 9, wherein the extraction step takes place at a temperature between 25 and 150 °C and over 1 to 5 extraction cycles.

11. A method for extracting cynaropicrin according to any one of claims 1-10, wherein the biomass/solvent ratio is between 1/4 and 1/100.

12. A method of extracting cynaropicrin according to any one of claims 2-11, wherein the ethanol/water ratio is 40/60.

## Patentansprüche

1. Verfahren zur Extraktion von Cynaropicrin aus Blättern von *Cynara cardunculus*, **dadurch gekennzeichnet dass**, es die folgenden Schritte umfasst:
- Lyophilisierung und Mahlen von Blättern von *Cynara cardunculus* mit einer Partikelgröße von weniger als 400 µm;
- Auswahl eines Lösungsmittels aus Ethanol oder Ethylacetat in reinem Zustand oder in einer Mischung;
- Extraktion der lyophilisierten Biomasse mit dem im vorherigen Schritt ausgewählten Lösungsmittel in einem geschlossenen Behälter und vor Licht geschützt.

2. Verfahren zur Extraktion von Cynaropicrin nach Anspruch 1, wobei das Lösungsmittelgemisch ein Ethanol / WasserGemisch ist.

3. Verfahren zur Extraktion von Cynaropicrin nach einem der Ansprüche 1 oder 2, wobei in dem Extraktionsschritt der Behälter der Wirkung eines Bades oder eines Sondenultraschallgeräts ausgesetzt wird.

4. Verfahren zur Extraktion von Cynaropicrin nach einem der Ansprüche 3, wobei der Extraktionsschritt eine Dauer zwischen 15 und 240 Minuten hat, bei einer Temperatur zwischen 25 und 60 ° C und bei einer Ultraschallleistung zwischen 5 und 100 kHz stattfindet.

5. Verfahren zur Extraktion von Cynaropicrin nach einem der Ansprüche 1 oder 2, wobei in dem Extraktionsschritt der Behälter der Wirkung einer Rühreinrichtung ausgesetzt wird

6. Verfahren zur Extraktion von Cynaropicrin nach Anspruch 5, wobei der Extraktionsschritt eine Dauer zwischen 15 und 240 Minuten hat, bei einer Temperatur zwischen 25 und 60 ° C und unter Rühren zwischen 300 und 900 Umdrehungen pro Minute stattfindet.

7. Verfahren zur Extraktion von Cynaropicrin nach einem der Ansprüche 1 oder 2, wobei in dem Extraktionsschritt der Behälter der Wirkung einer Mikrowellenausrüstung ausgesetzt wird.

8. Verfahren zur Extraktion von Cynaropicrin nach Anspruch 7, wobei der Extraktionsschritt eine Dauer zwischen 5 und 120 Minuten hat, bei einer Temperatur zwischen 25 und 80 ° C und bei einer Mikrowellenleistung zwischen 500 und 2000 W stattfindet.

9. Verfahren zur Extraktion von Cynaropicrin nach einem der Ansprüche 1 oder 2, wobei in dem Extraktionsschritt der Behälter der Wirkung einer unter Druck stehenden Flüssigkeitsextraktionsausrüstung ausgesetzt wird.

10. Verfahren zur Extraktion von Cynaropicrin nach Anspruch 9, wobei der Extraktionsschritt bei einer Temperatur zwischen 25 und 150 ° C und über 1 bis 5 Extraktionszyklen stattfindet.

11. Verfahren zur Extraktion von Cynaropicrin nach einem der Ansprüche 1 bis 10, wobei das Verhältnis von Biomasse zu Lösungsmittel zwischen 1/4 und 1/100 liegt.

12. Verfahren zur Extraktion von Cynaropicrin nach einem der Ansprüche 2 bis 11, wobei das Verhältinis von Ethanol / Wasser 40/60 beträgt.

## Revendications

1. Procédé pour l'extraction de cynaropicrine à partir de feuilles de *Cynara cardunculus*, **caractérisé en ce qu'**il comprend les étapes suivantes :
- lyophilisation et broyage des feuilles de *Cynara cardunculus*, avec une granulométrie inférieure à 400 µm ;
- la sélection d'un solvant parmi l'éthanol ou l'acétate d'éthyle à l'état pur ou en mélange ;
- extraction de la biomasse lyophilisée avec le solvant sélectionné à l'étape précédente dans un récipient fermé et protégé de la lumière.

2. Procédé d'extraction de cynaropicrine selon la revendication 1, dans lequel le mélange de solvants est un mélange éthanol/ eau.

3. Procédé pour l'extraction de cynaropicrine selon l'une quelconque des revendications 1 ou 2, dans lequel dans l'étape d'extraction le récipient est soumis à l'action d'un bain ou d'un équipement de sonde à ultrasons.

4. Procédé pour l'extraction de la cynaropicrine selon l'une quelconque des revendications 3, dans lequel l'étape d'extraction a une durée comprise entre 15 et 240 minutes, se déroule à une température comprise entre 25 et 60 °C et à une puissance ultrasonore comprise entre 5 et 100 kHz.

5. Procédé pour l'extraction de cynaropicrine selon l'une quelconque des revendications 1 ou 2, dans lequel dans l'étape d'extraction le récipient est soumis à l'action d'un équipement d'agitation.

6. Procédé pour l'extraction de cynaropicrine selon la revendication 5, dans lequel l'étape d'extraction a une durée comprise entre 15 et 240 minutes, se déroule à une température comprise entre 25 et 60 °C et avec une agitation comprise entre 300 et 900 rotations par minute.

7. Procédé pour l'extraction de cynaropicrine selon l'une quelconque des revendications 1 ou 2, dans lequel dans l'étape d'extraction le récipient est soumis à l'action d'un équipement à micro-ondes.

8. Procédé pour l'extraction de cynaropicrine selon la revendication 7, dans lequel l'étape d'extraction a une durée comprise entre 5 et 120 minutes, se déroule à une température comprise entre 25 et 80 °C et à une puissance de micro-ondes comprise entre 500 et 2000 W.

9. Procédé pour l'extraction de cynaropicrine selon l'une quelconque des revendications 1 ou 2, dans lequel dans l'étape d'extraction le récipient est soumis à l'action d'un équipement d'extraction de liquide sous pression.

10. Procédé pour l'extraction de cynaropicrine selon la revendication 9, dans lequel l'étape d'extraction se déroule à une température comprise entre 25 et 150°C et sur 1 à 5 cycles d'extraction.

11. Procédé pour l'extraction de cynaropicrine selon l'une quelconque des revendications 1 à 10, dans lequel le rapport biomasse/ solvant est compris entre 1/4 et 1/100.

12. Procédé pour l'extraction de cynaropicrine selon l'une quelconque des revendications 2-11, dans lequel le rapport éthanol/ eau est de 40/60.
